# EUROPEAN PATENT APPLICATION

(11) **EP 1 424 338 A1**
(43) Date of publication of application: **02.06.2004**
(21) Application number: 02774250.1
(22) Date of filing: 03.09.2002
(51) Int. Cl.: C07D 473/40

(54) **A PROCESS FOR PREPARING 2,6-DICHLOROPURINE**

(30) Priority: 07.09.2001 CN 01142068
(71) Applicant: ZHEJIANG CHENGYI PAHRMACEUTICAL CO.LTD, Dongtou County, Zhejiang 325700 (CN)
(72) Inventor: YAN, Yiyi No.118 Huagong Road, Zhejiang 325700 (CN); CEN, Junda Room 1001, No.25, Lane 465, Shanghai 200434 (CN)
(74) Representative: Rupp, Christian, Dipl.Phys.
(86) International application number: PCT/CN2002/000603
(87) International publication number: WO 2003/048161

(57) **Abstract**

A process for easily and efficiently preparing 2,6-dichloropurine. In this process 2,6-dichloropurine is obtained in one step diazotization using 2-amino-6-chloropurine as starting material in the presence of chloride or hydrochloric acid.

## Description

### FIELD OF THE INVENTION

The present invention relates to the organic chemistry field, and in particular, the invention provides a process for preparing 2,6-dichloropurine.

### BACKGROUND OF THE INVENTION

2,6-dichloropurine is an important intermediate in synthesizing nucleoside compounds such as the anti-cancer drug―Fludarabine. It is of great value in the use in medical chemical industry.

Various processes to obtain 2,6-dichloropurine have been disclosed in the prior art, e.g. by nitrification, chlorination, reduction, and cyclization using 4-amino-2,6-dihydroxypyrimidine as starting material (Bitterli P, Helv.Chim.Acta.1951,34:834). This process lacks the value for practical usage due to long steps and low total yield wherein the yields of chlorination and cyclization are only 32% and 51% respectively.

The intermediate also is manufactured by chlorination. One method uses hypoxanthine 1-N-oxide as starting material, (Kavashima H, Bull. Chem. Soc.Jpn 1967,40:639). However, it is difficult to obtain the starting material.

Another chlorination method uses 2,6-dimercaptopurine as starting material, (Singh PK, Indin J. Chem. 1986, 25B(8):823). This second chlorination method lacks industrial production value due to the elaborate steps required, and the generation of severe pollution. Also, the use of chlorine presents a safety hazard.

European Patents EP 543095 and EP433846 teach a process for preparing the starting material, 2-amino-6-chloropurine.

Therefore, there is a need for an easy and efficient process for preparing 2,6-dichloropurine. The process should be scalable to industrial production quantities.

### SUMMARY OF THE INVENTION

The object of the present invention is to overcome the defects existing in the prior art, and to provide an easy and economic process for preparing 2,6-dichloropurine which is suitable for industrial production.

### DETAILED DESCRIPTION OF THE INVENTION

According to the present invention, 2,6-dichloropurine( I ) is obtained by using 2-amino-6-chloropurine(II)as starting material in a one step diazotization. The scheme of reaction is as follows:

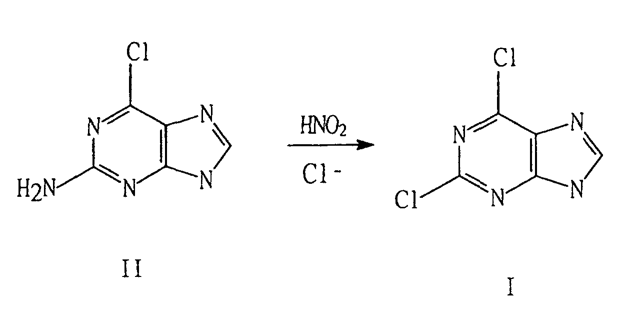

The process according to the present invention includes obtaining 2,6-dichloropurine by reacting 2-amino-6-chloropurine with a nitric acid in the presence of a chloride. The nitric acid is obtained by reacting a nitrite with an acid.

In particular, the process includes the following steps: 2-amino-6-chloropurine is added into an acid solution containing a chloride, the mixture is cooled afterward, and then a nitrite is added into the mixture. 2,6-dichloropurine is obtained by diatizodation.

The chloride utilized is an aqueous solution of the chloride such as sodium chloride, potassium chloride, zinc chloride, calcium chloride, lithium chloride, magnesium chloride, aluminum chloride or ammonium chloride.

The acid utilized is an inorganic acid such as hydrochloric acid, sulfuric acid or phosphoric acid, and preferably is hydrochloric acid. The nitrite is sodium nitrite or potassium nitrite, and preferably is sodium nitrite.

The reaction temperature is -50 to +50°C, and preferably is -10 to +10°C; the reaction time 10 to 150 minutes, and preferably is 20 to 60 minutes.

The reaction is followed by the extraction by a solvent, wherein the solvent is an organic solvent indissoluble in water, such as diethyl ether, diisopropyl ether, ethyl acetate, n-butyl acetate and toluene. A preferred solvent is ethyl acetate.

Surprisingly and unexpectedly, the inventor found through investigation that the reaction described above can even be carried out in the presence or absence of a chloride if hydrochloride is used as an inorganic acid. However, the yield of 2,6-dichloropurine can be greatly improved by use of a chloride. Thus, the present invention also provides a process for preparing 2,6-dichloropurine, comprising reacting 2-amino-6-chloropurine with a nitric acid in hydrochloride acid, wherein the nitric acid is obtained by reacting hydrochloric acid with a nitrite.

In the process of the invention, 2,6-dichloropurine is obtained in one step of diazotization by using easily available materials. As such, the process is highly valuable in industrial production due to short-term reaction, easy operation, and a high yield.

The invention will hereinafter be described by way of the following examples. It should be understood that, the process in the examples of the invention are description of the invention, but not a limit to the invention. Simple modification of the process according to the invention under the spirit of the invention all fall into the scope of the appended claim.

### Example 1: preparation of 2,6-dichloropurine (in the presence of chloride)

400 ml concentrated hydrochloric acid is poured into a flask, cooled in water bath and stirred. 300g zinc chloride is added slowly and the mixture is cooled down to 10°C. 100g 2-amino-6-chloropurine is added and the mixture is cooled to -5°C. 55g sodium nitrite is added in portions at a temperature kept below 5°C over about 0.5 hour.

The mixture is continually stirred for 0.5 hour and then 500ml water is added into the mixture. The mixture is extracted with ethyl acetate (500ml × 4), the organic phase is washed with water (300ml × 2), and then all ethyl acetate is recovered. 75g 2,6-dichloropurine was obtained by recrystallization using methanol. The product is a white needle crystal and has a melting point of 180-182 °C. Yield is 67%.

### Example 2: preparation of 2,6-dichloropurine (in the absence of chloride)

400 ml concentrated hydrochloric acid is poured into a flask, stirred and cooled in ice-water bath to 10°C. 100g 2-amino-6-chloropurine is added and the mixture is cooled to -5°C. 55g sodium nitrite is added in portions at a temperature kept below 5 °C over about 0.5 hour. The mixture is continually stirred for 0.5 hour and then 500ml water is added into the mixture. The mixture is extracted with ethyl acetate(500ml × 4) and the organic phase is washed with water(300ml × 2), then all ethyl acetate is recovered.

40g 2,6-dichloropurine was obtained by recrystallization using methanol. The product is a white needle crystal and has a melting point of 180-182 °C. Yield is 36%.

While the invention has been described with reference to details of the illustrated embodiment, these details are not intended to limit the scope of the invention as defined in the appended claims.

## Claims

1. A method for preparing 2,6-dichloropurine or the acidic addition salt thereof, 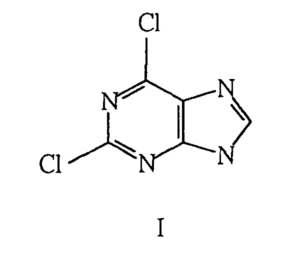 the process comprising reacting 2-amino-6-chloropurine with nitric acid in the presence of chloride.

2. The method as recited in claim 1, wherein the nitric acid is obtained by reacting a nitrite with an acid.

3. The method as recited in claim 1, wherein the chloride is an aqueous chloride selected from the group consisting of sodium chloride, potassium chloride, zinc chloride, calcium chloride, lithium chloride, magnesium chloride, aluminum chloride, and ammonium chloride.

4. The method as recited in claim 2, wherein the acid is hydrochloric acid, or sulfuric acid or phosphoric acid.

5. The method as recited in claim 2, wherein the nitrite is sodium nitrite or potassium nitrite.

6. The method as recited in claim 1, wherein the reacting step occurs at a temperature of -50 to +50°C.

7. The method as recited in claim 6, wherein the temperature is -10 to 10°C.

8. A method for preparing 2,6-dichloropurine or the acidic addition salt thereof, 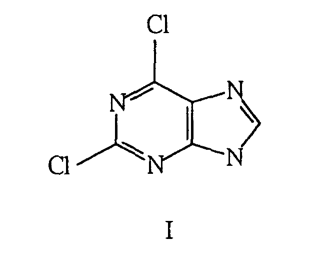 the process comprising reacting 2-amino-6-chloropurine with a nitric acid in the presence of hydrochloric acid.

9. The method as recited in claim 8, wherein the nitric acid is obtained by reacting hydrochloric acid with nitrite.

10. The method as recited in claim 9, wherein the nitrite is sodium nitrite or potassium nitrite, and the reacting step occurs at -10 to 10°C.
